(19) Europäisches Patentamt
European Patent Office
Office européen des brevets

(11) Publication number: **0 513 434 A1**

# (12) EUROPEAN PATENT APPLICATION

(21) Application number: **91117064.5**

(51) Int. Cl.5: **C07D 239/42**, A61K 31/505

(22) Date of filing: **07.10.91**

(30) Priority: **15.05.91 CS 1426/91**

(43) Date of publication of application:
**19.11.92 Bulletin 92/47**

(84) Designated Contracting States:
**AT BE CH DE FR GB IT LI**

(71) Applicant: **VYZKUMNY USTAV PRO FARMACII A BIOCHEMII STATNI PODNIK**
**Kourimska 17**
**CS-130 60 Praha 3(CS)**

(72) Inventor: **Kejha, Jiri, Dr.**
**V zahradach 20/878**
**CS-180 00 Praha 8(CS)**
Inventor: **Brunova, Bohumila**
**Ostrovského 33**
**CS-150 00 Praha 5(CS)**
Inventor: **Slukova, Darina**
**Celedova 1785**
**CS-149 00 Praha 4(CS)**
Inventor: **Kuchar, Miroslav**
**Ambrozova 14**
**CS-130 00 Praha 3(CS)**
Inventor: **Knezova, Eva, Dr.**
**Makovského 1206**
**CS-163 00 Praha 6(CS)**
Inventor: **Grimova, Jaroslava, Dr.**
**Na Doubkové 2**
**CS-150 00 Praha(CS)**

(74) Representative: **von Füner, Alexander, Dr. et al**
**Patentanwälte v. Füner, Ebbinghaus, Finck**
**Mariahilfplatz 2 & 3**
**W-8000 München 90(DE)**

## (54) 5-//4-/(4',6'-dimethyl-2-pyrimidinyl)sulfamoyl/phenyl/azo/acetylsalicylic acid (DISALAZINE).

(57) This invention relates to 5-//4-/(4',6'- dimethyl-2-pyrimidinyl)sulfamoyl/phenyl/azo /acetylsalicyclic acid (DISALAZINE) with the production via diazonium salt of sulf adimidine reacted with salicylic acid and then is acetylated. Claimed substance has anti-arthritic and immunomodulating properties.

EP 0 513 434 A1

The substance which is the subject of the invention is an analogue of a known therapeutic, namely sulfasalazine (5-//4-/(4',6'- dimethyl-2-pyrimidinyl)sulfamoyl/phenyl/azo/acetylsalicylic acid) discovered in 1942 (Acta Medica Scandinavica 60, 1942, p. 577 - 598, N. Schwartz) and used in the therapy of progressive polyarthritis and ulcerative colitis. Sulf asalazine also influences various steps in the metabolism of arachidonic acid and has immunomodulating properties (J.Clin. Invest. 69, 1982, p. 494, W.F.Stenson). A shortcoming of this drug is its low biological availability and some unfavorable side effects. Therefore a series of analogues of sulf asalazine was synthesized with the aim to find a substance of more favorable therapeutic and pharmacokinetic properties, especially a higher biological availability.

5-//4-(4',6'- dimethyl-2-pyrimidinyl)sulfamoyl/phenyl/azo/acetylsalicylic acid DISALAZINE of the following formula I

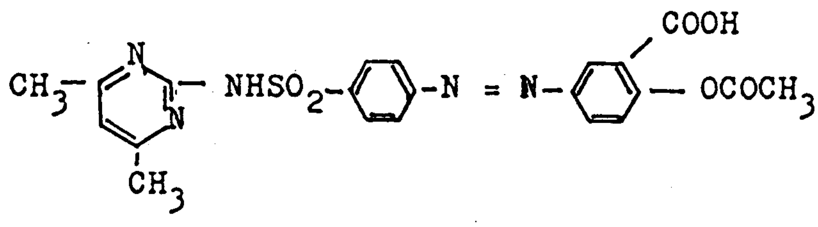

(I)

and its production are accomplished by first preparing the diazonium salt of sulfadimidine (4,6-dimethyl-2-sulfanilamidopyrimidine) by a known process and is then left to react at from -2 °C to + 10 °C with salicylic acid in an alkaline medium, e.g. sodium hydroxide, sodium carbonate and the like. The resulting product is precipitated by acidification and is collected on a filter. The product must be precipitated, e.g. from a $NaHCO_3$ solution mainly to remove mechanical impurities as well as other extraneous products. For the acidification of the alkaline solution it is advantageous to add ethyl acetate to precipitate the crystalline product. In the next step acetylation of the hydroxy group is carried out with the aid of acetanhydride or acetylchloride, at the presence of toluene on acetic acid. Otherwise the diazonium salt of sulfadimidine can be reacted directly with acetylsalicylic acid in an alkaline medium.The resulting product precipitates upon acidification and is collected on a filter.

Experimentally the substance has a significant anti-arthritic and immunomodulating activity, low toxicity, and three times greater biological availability in comparison with sulfasalazine.

It is possible to use the claimed substance as a therapeutic drug with antiarthritic and immunomodulating activity.

The antiarthritic activity of DISALAZINE was tested in comparison with sulfasalazine in a model of rat adjuvant disease with preventive administration.The clinical symptoms of disease (oedema of limbs,osteous lesions,articular motility,body weight), as well as its influence on the immunological functions was evaluated.DISALAZINE significantly diminished the size of limbs oedema, the extent of osteous injury and increased the articular motility and the body weight.In all instances,DISALAZINE gave better results than sulfasalazine (Table 1).

Table 1 Antiarthritic activity of DISALAZINE(dose 400 mg/kg)%

| | body weight | limb oedema | articular motility | osteous lesions |
|---|---|---|---|---|
| Healthy rats | 100 | 0 | 100 | 0 |
| Non-medicated rats | 35 | 100 | 31 | 100 |
| Medicated rats by: | | | | |
| DISALAZINE | 68 | 52 | 80 | 27 |
| sulfasalazine | 37 | 57 | 53 | 66 |

Neither DISALAZINE and sulfasalazine in the dose of 400mg/kg did not affected the number of exsudate cells in the model of experimental pleuritis.The phagocytic activity (the number of phagocyting cells) measured on the model of phagocytosis of peritoneal macrophages was significantly reduced by DISALAZINE (49.6 % of control) like as sulfasalazine (54,7 % of control),both in 400mg/kg : the cellularity of exsudate was reduced to 55 % of control,similarly as the number of glas adherent cells (52 % of control).

The immunosupression induced by azathioprine was significantly inhibited by DISALAZINE after multiple doses of 100 mg/kg. DISALAZINE showed expressive in vitro effect on lymphocyte proliferation

induced by Con A.In comparison with sulfasalazine which had low effect,DISALAZINE in the concentration range 40 µg to 4 pg significantly stimulated lymphocyte proliferation.The stimulation of LPS induced proliferation of lymphocytes was milder and comparable with sulfasalazine.

The synthesis of LT $B_4$ was not affected by DISALAZINE in rat pleural PMNL.

Pharmacokinetic studies with labelled substances show that DISALAZINE in comparison with sufasalazine is substantially better resorbed, has a longer half-life of elimination and maximum blood levels are attained about 12 hours after administration.

The main advantage of the claimed substance is its experimentally proven significant anti-arthritic and immunomodulating activity and low toxicity. These properties give hope for its exploitation in clinical practice especially in the therapy of progressive polyarthritis. (See the Table 1.)

Details of the synthesis are obvious from the following examples which illustrate but do not in the least limit the whole process.

Example 1

50 g 4,6-dimethyl-2-sulfanilamidopyrimidine are dissolved in 250 ml water and 45 ml concentrated hydrochloric acid. The solution is cooled to 0°C - 5°C and a solution of 12 g $NaNO_2$ in 50 ml water is added in drops. It is stirred for 1 h yet at the same temperature and the solution of the diazonium salt is then slowly added to 25.2 g salicylic acid dissolved in a solution of 30 g NaOH and 250 ml water. The resulting red-brown solution is then stirred for 30 minutes. Afterwards 300 ml ethyl acetate is added and under intensive stirring 70 ml 1 : 1 dilute hydrochloric acid is added drop by drop. The precipitated substance is collected on a filter and dried at 110°C. 70.4 g, i.e. 91.6 % th. 5-//4-/(4',6'-dimethylpyrimidinyl )sulfamoyl/phenyl/azo/salicylic acid with melting point of 226°C - 231°C is isolated.

Example 2

85.5 g 5-//4-(4',6'-dimethylpyrimidinyl)sulfamoyl/phenyl/azo/salicylic acid is dissolved in a solution prepared by dissolving 33.6 g $NaHCO_3$ in 100 ml water. The resulting alkaline aqueous solution is extracted with 250 ml ethyl acetate. The water phase is filtered with charcoal and after the addition of 366 ml of ethyl acetate the alkaline solution is acidified with hydrochloric acid diluted 1:1 until an acid reaction is obtained. The precipitated product is collected on a filter and washed in water. 82.8 g refined 5-//4-(4',6'-dimethylpyrimidinyl)sulfamoyl/phenyl/azo/salicylic acid with m.p.228-230°C is isolated.

Example 3

20 g 5-//5-/(4',6'-dimethylpyrimidinyl)sulfamoyl/phenyl/azo/salicylic acid is suspended in a mixture of 10 ml acetanhydride, 20 ml acetic acid, and 0.05 g concentrated sulphuric acid. The reaction mixture is heated to 130°C - 150°C and stirred at this temperature for 2 h. Upon cooling to 100°C20 ml toluene is added and stirring continues for a further 1 h. After cooling down the precipitated product is collected on a filter and dried at 110°C - 120°C. 17.5 g 5-//4-/(4',6'-dimethyl-2-pyrimidinyl)sulfamoyl/phenyl/azo/acetylsalicylic acid, i.e. 80 % th. with a melting point of 185°C - 187°C (decomposed) is is isolated.The structure of the recovered substance has been confirmed by emission absorbance (EA) and NMR.

Example 4

11.6 g 4,6-dimethyl-2-sulfanilamidopyrimidine is dissolved in a mixture of 140 ml water and 11 ml concentrated hydrochloric acid, the solution is cooled to 0°C - 5°C and within 20 minutes 3 g $NaNO_2$ in 140 ml water is added drop by drop. It is stirred further for 1 h at the some temperature and the solution of the diazonium salt is then slowly poured to a solution of 7.7 g acetylsalicylic acid in 220 ml water and 10.7 g sodium hydroxide cooled to 10°C. The reaction mixture is stirred yet for 3 h at room temperature. Precipitated crystals of the sodium salt are collected on a filter. Still moist, they are dissolved in 200 ml water and the solution is acidified with hydrochloric acid. The precipitate is collected on a filter, washed 5 times in 100 ml water and dried at 110°C - 120°C. 12.3 g, i.e. 62,9 % theory of 5-//4-/(4',6'-dimethyl-2-pyrimidinyl)sulfamoyl/phenyl/azo/acetylsalicylic acid with melting point of 186°C(decomposed) is isolated.

Example 5

21.4 g 5-//4-/(4',6'-dimethylpyrimidinyl)sulfamoyl/phenyl/-azo/salicylic acid is suspended in a mixture of

13.27 g acetanhydride and 17.1 ml toluene. The reaction mixture is heated under stirring 5 h. After cooling to 20°C solid fraction is filtered and washed with toluene. After drying is 21.3 g 5-//4-/(4', 6'-dimethy1-2-pyrimidinyl)sulfamoyl/phenyl/azo/acetylsalicylic acid,i.e. 90,6% th.,which is recrystalized from acetanhydride with the yield 75-80% th.

## Claims

**1.** 5-//4-/(4',6'-dimethyl-2-pyrimidinyl)sulfamoyl/phenyl/azo/acetylsalicylic acid, i.e. DISALAZINE of formula (I)

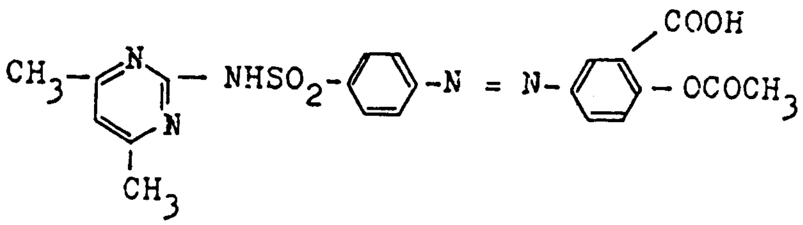

(I)

**2.** A process for preparing the compound according to claim 1, characterized in that a diazonium salt of sulfadimidine is reacted with salicylic acid in an alkaline medium and upon acidification in the presence of ethyl acetate the product is isolated and reprecipitated via the sodium salt and in the next step the hydroxy group of salicylic acid is acetylated.

**3.** A process according to claim 1, characterized in that a diazonium salt of sulfadimidine is reacted directly with acetylsalicylic acid in an alkaline medium.

European Patent Office

# EUROPEAN SEARCH REPORT

Application Number

EP 91 11 7064

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | ACTA MEDICA SCANDINAVICA<br>vol. 110, no. 6, 1942, STOCKHOLM<br>pages 577 - 598;<br>N. SVARTZ: 'SALAZOPYRIN, A NEW SULFANYLAMIDE PREPARATION'<br>* page 577 - page 579 *<br>----- | 1,3 | C07D239/42<br>A61K31/505 |

| TECHNICAL FIELDS SEARCHED (Int. Cl.5 ) |
|---|
| C07D |

The present search report has been drawn up for all claims

| Place of search | Date of completion of the search | Examiner |
|---|---|---|
| THE HAGUE | 30 JUNE 1992 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)